# EUROPEAN PATENT APPLICATION

(11) **EP 2 431 038 A1**
(43) Date of publication of application: **21.03.2012**
(21) Application number: 10772313.2
(22) Date of filing: 07.04.2010
(51) Int. Cl.: A61K 31/495, A61K 9/127, A61P 31/16

(54) **PHARMACEUTICAL COMPOSITION CONTAINING ARBIDOL IN THE FORM OF PHOSPHOLIPID NANOPARTICLES**

(30) Priority: 05.05.2009 RU 2009116832
(71) Applicant: Obchshestvo S Ogranichennoy Otvetstvennostyu <<Ekobiofarm>>, Moscow 107078 (RU)
(72) Inventor: ARCHAKOV, Alexandr Ivanovich, Moscow 117321 (RU); GUSEVA, Mariya Kirillovna, Moskovskaya obl. 141400 (RU); UCHAYKIN, Vasily Fedorovich, Moscow 117420 (RU); IPATOVA, Olga Mikhaylovna, Moscow (RU); DOCHSHITSIN, Yury Fedorovich, Moscow 123100 (US); TIKHONOVA, Yelena Georgiyevna, Moscow 105064 (RU); MEDVEDEVA, Natalya Velorikovna, Moscow 119517 (RU); PROZOROVSKY, Vladimir Nikolayevich, Moscow 117526 (RU); STREKALOVA, Oksana Sergeevna, Moscow (RU); SHIRONIN, Alexandr Vladimirovich, Moscow 129090 (RU)
(74) Representative: Einsel, Martin
(86) International application number: PCT/RU2010/000163
(87) International publication number: WO 2010/128889

(57) **Abstract**

The invention relates to the fields of medicine and pharmacology and concerns a storage-stable composition made up of nanoparticles which are based on vegetable phospholipids and contain the antiviral drug Arbidol. The aim of the present invention is to develop a non-fatty phospholipid composition of Arbidol in which the micellar and liposomal particles have an average diameter of 8-25 nm, and which has low toxicity, can be stored for long periods and is capable of transporting the medicinal agent in the body and ensuring the high bioaccessibility of said agent. This aim is achieved by a phospholipid composition of Arbidol in the form of phospholipid nanoparticles with a size of 8-25 nm, which contains phosphatidylcholine, maltose and Arbidol in the following component ratio: 20-43 mass% phosphatidylcholine; 55-78 mass% maltose; 2-8 mass% Arbidol.

## Description

### FIELD OF THE INVENTION

The invention relates to medicine and pharmacology, particularly storage-stable composition comprising nanoparticles that are based on vegetable phospholipids and contain the Arbidol antiviral drug.

### BACKGROUND OF THE INVENTION

Developing drugs possessing high bioaccessibility to reach the affected area of the organism upon administration is among priorities of modern pharmacology. Bioaccessibility of drugs depends, among many other factors, on their solubility.

Nearly 60% of pharmaceutics at the development stage and many drugs in wide use already are poorly soluble compounds.

It is common knowledge that a majority of medicines, including Arbidol, penetrate slowly, and in limited quantities, into cells through the natural barrier - their biological membrane. A drug is to have a certain lipophilic property to penetrate this barrier.

Over the recent decade, researchers have given much attention to both a search for new biologically active substances and also ways to improve the efficiency of existing drugs by developing systems for transporting them in the organism, and improving their bioaccessibility and the efficiency of their specific performance.

In this respect, a significant advantage is shown by phospholipid nanoparticles that are effective largely because of their size ranging from 15 to 25 nm. Phospholipid particles (liposomes and micelles) are biodegradable, biologically inert, and do not cause allergic, antigenic, or pyrogenic reactions. In contrast to other particles, the surface of lipid nanoparticles is easily modified for targeted delivery.

The efficiency of phospholipid nanoparticles as colloidal drug carriers is attributable essentially to their small dimensions, preferably from 15 to 25 nm, that give them a large effective surface and, as a result, a high capacity.

Because of their chemical structure, phospholipid nanoparticles can serve as carriers for medicines either soluble or insoluble (hydrophobic) in biological liquids. Medical compounds embedded into the lipid matrix of nanoparticles helps obtain new drug nanoforms that have a high efficiency and bioaccessibility and low side effects.

In combination with nano-size, the large total surface area produces optimal conditions for reaction between such particles and the cell. Furthermore, nano-size offers a unique opportunity for particles to be embedded in the area of intercellular slit contacts that may be 30 to 50 nm at some points. As a result, medicinal agents can be delivered to areas of affected, for example, inflamed, tissue inaccessible to other medicines.

Together with the common surface pattern, the almost similar dimensions of phospholipid nanoparticles and lipoproteins create optimal conditions for reaction between them as well. In particular, drug-transporting phospholipid particles are incorporated into the system of blood plasma lipoproteins and lipid-transporting proteins and, as a result, lipophilic drug molecules can, together with phospholipids, be transported to lipoprotein particles.

A prior art publication describes dispersed micro particles stabilized by phospholipid in a fast-dispersing solid dosed form that consists of a water-insoluble compound in the form of nano-sized or micro-sized solid particles having their surface stabilized by surface modifiers, for example, by phospholipid, the particles being dispersed in a volume-producing matrix (Russian Patent No. 2,233,654). The resultant particles have a size of 0.66 to 10.6 µm (660 to 10,000 nm).

A prior art method for producing submicron particles of an organic pharmaceutically active compound that is not, or is poorly, dissolved in water comprises dissolving the compound in a first solvent mixed with water, mixing the solution with a second solvent, with phosphatidylcholine added thereto, and homogenizing the same, or homogenizing the resultant presuspension in a reflux flow, or subjecting it to ultrasound (Russian Patent No. 2,272,616). The resultant particles have a size of 0.1 to 2.46 µm (100 to 2,500 nm).

European Patent EP 0556394 A1 discloses a method for producing a lyophilized preparation for delivering medicines on the basis of refined soy oil and refined egg phosphatidylcholine. The preparation is dissolved easily in water, producing particles of 10 to 100 nm in size, and is a fatty emulsion.

A well-known antiviral medicine, Arbidol, has the chemical name of ethyl ester of 6-bromo-5-hydroxy-1-methyl-4-dimethylaminomethyl-2-phenylthiomethylindole-3-carbonic acid hydrochloride monohydrate, described by the chemical formula: C₂₂H₂₅Br N₂O₃S·HCl (Russian Drug Register. Encyclopedia of Medicines, 7th edition, 2000, p. 95).

Arbidol is a crystalline powder of greenish-white to greenish-light yellow color. It is practically insoluble in water.

Arbidol specifically inhibits flu A and B viruses. It displays its antiviral effect by suppressing the merger of the lipid membrane of the virus with cell membranes upon contact between the virus and cell. Its medical efficiency against flu shows up in reduced intoxication symptoms, lower intensity of catarrhal phenomena, and shorter period of fever and total duration of sickness. It prevents development of post-flu complications, decreases the frequency of attacks of chronic sicknesses, and normalizes immunological indexes.

When used internally, it is digested from the gastrointestinal tract and delivered to organs and tissues. The Cₘₐₓ in the blood at a 50 mg dose is reached within 1.2 hour, within 1.5 hour at a 100 mg dose, and T₁/2 in approximately 17 hours. It is excreted unchanged with urine and feces.

### SUMMARY OF THE INVENTION

It is an object of the present invention to develop a nonfatty phospholipid Arbidol composition having an average diameter of liposomal-micellar particles between 8 and 25 nm in size and a low toxicity, capable of remaining in storage for a long time period, and transporting a medicinal agent in the organism at a high bioaccessibility rate.

The object of the invention is achieved by using a phospholipid Arbidol composition in the form of phospholipid nanoparticles having a size of 8 to 25 nm, said composition comprising phosphatidylcholine, maltose, and Arbidol at the following ratio of the components, in mass %:

| | |
|---|---|
| phosphatidylcholine | 20-43 |
| maltose | 55-78 |
| Arbidol | 2-8. |

Phosphatidylcholine used in the invention is the principal component of highly refined vegetable soy phospholipid that contains at least 73 to 95 mass % of phosphatidylcholine. It may contain other phospholipid components in acceptable quantities (up to 4 mass % of lysophosphatidylcholine and trace quantities of other phospholipids).

The composition contains maltose as an auxiliary pharmacologically acceptable substance for producing lyophilizate capable of fully restoring its structure (specifically, particle size) following dissolution in water.

In accordance with the invention, the claimed composition is suitable for producing a pharmaceutical preparation that contains Arbidol having a greater bioacceptability than the original medicine.

### BRIEF DESCRIPTION OF THE DRAWINGS

FIG. 1 illustrates pharmacokinetics of Arbidol contained in phospholipid nanoparticles (NF Arbidol) and Arbidol following peroral administration of the preparations (at a dose of 25 mg/kg) in experiments on rats.
FIG. 2 shows an average lifetime of animals in the control group and in groups of mice given Arbidol and NF Arbidol daily at an Arbidol dose of 25 and 40 mg/kg of weight.
FIG. 3 (a and b) shows the extent of protection against mortality in percent on the sixth (a) and 15th (b) days (ratio of animals surviving at this date to the number of animals in the group).
FIG. 4 shows changes in the average weight of animals in the groups from infection time.

### AN EMBODIMENT OF THE INVENTION

The method for producing a phospholipid Arbidol composition is performed as follows:

### Materials and Techniques

The following materials were used to carry out the method:
1. Soy phospholipid containing 73% to 95% of phosphatidylcholine, from Lipoid GmbH, Germany.
2. Maltose monohydrate, from Merck, Germany.
3. Injection water (according to FS No. 42-4587-95).
4. Arbidol, substance (according to FS No. 42-3151-95).

### 1. Production of Arbidol contained in phospholipid nanoparticles (NF Arbidol)

### A. Preparation of a coarse emulsion:

1. 25 g of maltose was dissolved in 200 ml of water at a temperature of 45°C (until fully dissolved).
2. 625 mg of Arbidol and 6.25 g of phospholipid were added to the resultant maltose solution.
3. A domestic blender was used to homogenize the resultant mixture, and its volume was increased to 250 ml.

### B. Preparation of NF Arbidol:

1. The coarse emulsion was pumped through a homogenizer (Mini-Lab 7.3 VH, Rannie, Denmark) at a pressure of 800 bar.
2. Optical transmission was registered at 660 nm.
3. The preparation was filtered through a 0.45 micron mesh filter.
4. Optical transmission was registered again at 660 nm.
5. The size of particles in the preparation was measured.
6. The content of Arbidol in the preparation produced was analyzed by high-performance liquid chromatography.
7. The preparation was emptied into 10 ml flasks and dried lyophilically.
8. The content of a 10 ml flask was dissolved and the size of particles obtained by dissolution of lyophilically dried preparation powder was determined.
9. The size of particles was determined a day, two days, and a week after the dissolved preparation was stored at a temperature of 4°C.

### Results

An analysis of the particle size in a Beckman NS tester (see: Table 1) showed that over 96% of the particles had a size of (9.1 ± 1.0) nm. Judging by the size of a phospholipid molecule (length of fatty acid trails), the resultant particles were micelles. The particle size of the dissolved preparation was unchanged through a week of storage at 4°C.

**Table 1**

| Distribution of Particles of Arbidol Prepared as Part of Nanophospholipid Particles by Size | | | | |
|---|---|---|---|---|
| Measurement number | Measurement range, nm | Particle distribution | | |
| | | Size, nm | Content, % | Standard deviation, nm |
| 1 | 3.0-450.0 | 9.3 | 96.19 | 1.2 |
| 2 | 3.0-450.0 | 8.3 | 96.50 | 1.1 |
| 3 | 3.0-450.0 | 10.3 | 97.35 | 1.8 |
| 4 | 3.0-450.0 | 8.6 | 96.99 | 2.6 |

According to the results of high-performance liquid chromatography, 1 µl of the NF Arbidol preparation contained 2.5 µg (± 5%) of Arbidol, that is, practically 100% of Arbidol was embedded into phospholipid nanoparticles. Understandably, since Arbidol is not soluble in water, it is fully embedded into phospholipid micelles.

After the lyophilically dried NF Arbidol preparation was dissolved, the pattern of particle distribution by size (predominance of particles not larger than 9 nm in size) and the size of the predominant particle fraction did not change - more than 96% of the particles had a size of (9.0 ± 1.0) nm. The particle size did not change after the dissolved preparation was stored for seven days at 4°C.

**Table 2**

| Distribution of Particles in the Arbidol Preparation Solution as Part of Nanophospholipid Particles by Size upon Discharge and after Seven-Day Storage | | | | | |
|---|---|---|---|---|---|
| Measurement number | Storage time | Measurement range, nm | Particle distribution | | |
| | | | Size, nm | Content, % | Standard deviation, nm |
| 1 | 2 | 3 | 4 | 5 | 6 |
| 1 | Upon discharge | 3.0-450.0 | 9.7 | 97.26 | 1.6 |
| 2 | - | - | 8.9 | 96.37 | 1.3 |
| 3 | - | - | 8.4 | 96.96 | 2.1 |
| 4 | - | - | 9.1 | 96.53 | 1.7 |
| | | | | | |
| 1 | 2 | 3 | 4 | 5 | 6 |
| 1 | Seven days | 3.0-450.0 | 9.8 | 96.21 | 1.5 |
| 2 | | - | 9.5 | 96.15 | 1.9 |
| 3 | | - | 9.3 | 95.98 | 1.2 |
| 4 | | - | 8.9 | 97.11 | 2.3 |

2. Determination of Arbidol and NF Arbidol in solution, in whole blood, and in plasma in experiments *in vitro* (calibration of the determination method by high-performance liquid chromatography and validation of the method to determine completeness of extraction by the preferred method of pretreatment of biological liquids - blood and plasma).

### Determination of Arbidol in free form and as part of phospholipid nanoparticles:

2.1. Free Arbidol in a methanol solution (Arbidol is not dissolved in water and is dissolved only slightly in ethanol) was analyzed by high-performance liquid chromatography in a system of water and 0.1 % of TFA-acetonitrile. 20 µl of the methanol solution analyzed was added to the column. Chromatography was conducted for 0-5 minutes at the acetonitrile gradient of 5% to 60%, or 5 to 10 minutes at the acetonitrile gradient of 60%. The sensitivity of the method was at least 0.5 µg/ml, and the method had a linear dependence within the range of 0 to 1,000 ng of Arbidol.
2.2. 90 µl of methanol was added to 10 µl of the NF Arbidol solution and the mixture was agitated vigorously. The resultant solution was applied to the column as in paragraph 2.1.
2.3. Arbidol concentration was based on the value obtained on the calibration curve, with 1 µg of Arbidol corresponding to the peak area of 2,032 units on the chromatogram.

### Determination of Arbidol in whole blood and blood plasma in an experiment in vitro

1. Blood was collected into a test tube filled with EDTA until the final EDTA concentration was 1 mg per 1 ml of blood.
2. Lyophilically dried NF Arbidol was dissolved in 5 ml of water (5 mg/ml of Arbidol).
3. A suspension of 5 mg/ml of Arbidol in water was prepared.
4. **A.** 50 µl of NF Arbidol (5 mg/ml of Arbidol) was added to 950 µl of whole blood.

**B.** 50 µl of Arbidol aqueous suspension was added to 950 µm of whole blood.
5. The mixtures **A** and **B** were thoroughly mixed and incubated at 37°C for 20 minutes.
6. A 100 µl aliquot of the solutions **A** and **B** was collected into eppendorfs (C ≥ 1.5 ml), 900 µl of methanol was added, agitated intensively for 3 to 5 minutes, centrifuged for 10 minutes at 6,000 g to cause sedimentation of proteins, and the liquid above the sediment was analyzed for Arbidol content by high-performance liquid chromatography.
7. The whole blood containing Arbidol (solutions **A** and **B)** was centrifuged to obtain plasma (for 10 minutes at 6,000 g).
8. 900 µl of methanol was added to 100 µl of plasma, agitated intensively for 3 to 5 minutes, centrifuged for 10 minutes at 6,000 g, and the liquid above the sediment was analyzed for Arbidol content by high-performance liquid chromatography.
9. The results obtained by extraction of whole blood and plasma by methanol were compared. Physiological solution was used instead of whole blood as control to analyze the completeness of extraction.

### Results and Conclusions:

1. Treatment with a nine-fold excess of methanol chosen as a method for pretreating whole blood and plasma allows Arbidol to be extracted from whole blood and plasma completely.
2. Unlike NF Arbidol, though, free Arbidol causes hemolysis if added to whole blood. NF Arbidol can, therefore, be used for intravenous injections. Arbidol is so far used in the form of capsules and tablets only.
3. The dose-dependent effect of Arbidol and NF Arbidol on blood hemolysis in experiments *in vitro* is to be tested very thoroughly.

### Comparison of bioaccessibility of Arbidol and NF Arbidol in peroral administration in experiments on animals

### Description of the experiment:

1. The content of an NF Arbidol flask was dissolved in 5 ml. (A)
2. An Arbidol solution was prepared at a concentration of 5 mg/ml (suspension in water). (B)
3. Feed was withdrawn from the animals, leaving them water only, 24 hours before the experiment, and the animals were weighed on the day of the experiment.
4. 1 ml of solution A and B was administered perorally to the rats.
5. Blood was collected at intervals of 5, 10, 15, 30, and 45 minutes into eppendorfs containing EDTA. After an eppendorf was filled, it was shaken intensively.
6. Blood treatment: 900 µl of methanol was added to 100 µl of blood and agitated intensively for 3 to 5 minutes. The mixture was centrifuged to cause sedimentation of proteins. It was then analyzed by high-performance liquid chromatography for presence of Arbidol in the samples.

### Results

The dose administered was 25 mg/kg.

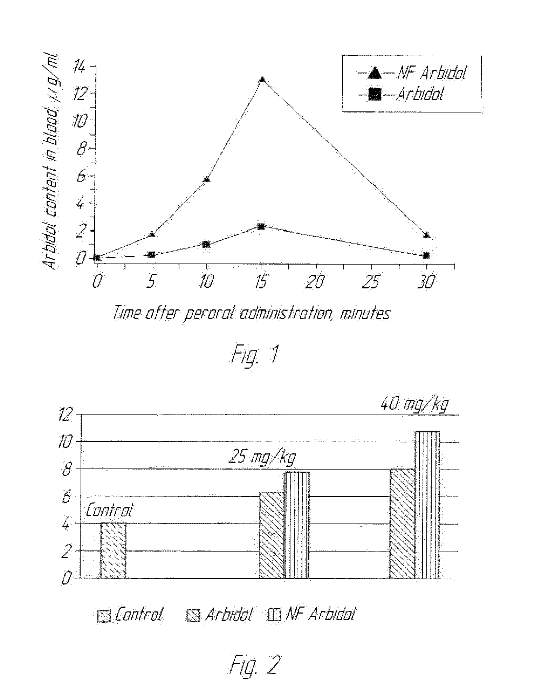

Table 3 and FIG. 1 show the test results of pharmacokinetics of Arbidol and NF Arbidol.

**Table 3**

| Time after administration, minutes | NF Arbidol (A), area/concentration in blood, µg/ml | Arbidol (B), area/concentration in blood, µg/ml |
|---|---|---|
| 5 | 7.1/1.75 | 0/0 |
| 10 | 22.8/5.61 | 1.2/0.3 |
| 15 | 53.1/13.1 | 7.69/2.4 |
| 30 | 8.12/2.0 | 1.6/0.4 |

### Comparison of antiviral activity of Arbidol and NF Arbidol on a model of influenza virus pneumonia in mice

Experiment scheme: The value of LD50 was determined in experiments conducted in advance in order to select an infection dose. Infection was then initiated with a virus dose at a multiple of 10 LD50. The mice were treated with two doses (25 mg/kg and 40 mg/kg of Arbidol) administered perorally every day, while the control group was given no treatment. Changes in animal weight, mortality protection factor, and average lifetime (in days), with Arbidol-containing medicines given daily, were selected as indicators of activity of the medicines studied in animals (mice) infected with virus AAii/2/69.

FIG. 2 shows comparative data for the average lifetime of the animals.

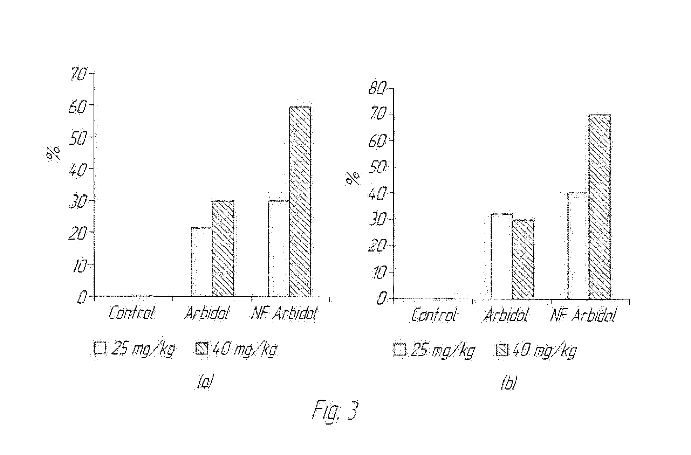

The drawing shows that treatment with Arbidol and NF Arbidol contributed to a longer average lifetime of the animals in comparison with the control group. Treatment with NF Arbidol, though, was more efficient than it was with Arbidol. Protection against mortality illustrated in FIG. 3 is also an efficiency factor of Arbidol-containing medicines.

Changes in the weight of animals in the experimental groups (FIG. 3) in relation to control (loss of weight is evidence of the severity of sickness) is a third criterion of antiviral efficiency of the medicines studied.

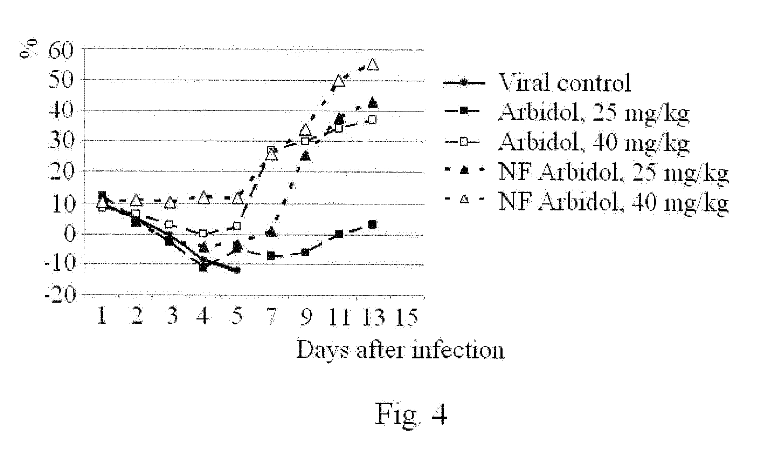

The diagram in FIG. 4 shows that maximum loss of weight in the control group occurred on the fifth day. No weight loss occurred in the groups of mice that received 40 mg/kg of NF Arbidol. Treatment with NF Arbidol, that is, with Arbidol embedded into phospholipid nanoparticles, was more effective than treatment with unbound medicine.

## Claims

1. pharmaceutical composition containing Arbidol in the form of phospholipid nanoparticles 8 to 25 nm in size, comprising 78% to 95% of vegetable phosphatidylcholine, maltose and Arbidol at the following ratio of the components, in mass %:
| | |
|---|---|
| phosphatidylcholine | 20-43 |
| maltose | 55-78 |
| Arbidol | 2-8. |
